# FASCICULE DE BREVET EUROPEEN

(11) **EP 3 416 604 B1**
(45) Date de publication et mention de la délivrance du brevet: **29.01.2020**
(21) Numéro de dépôt: 17710596.2
(22) Date de dépôt: 17.02.2017
(51) Int. Cl.: A61G 7/057, A61L 15/40, A61L 15/46, A61L 15/60, A47C 31/00, A61G 7/047

(54) **MATELAS POUR LA PREVENTION ET LE TRAITEMENT DES ESCARRES**
MATRATZE ZUR VERHINDERUNG UND BEHANDLUNG VON DEKUBITUS
MATTRESS FOR PREVENTING AND TREATING BEDSORES

(30) Priorité: 19.02.2016 FR 1670053
(43) Date de publication de la demande: 26.12.2018
(73) Titulaire: Wackermann, Guy, 67700 Monswiller (FR)
(72) Inventeur: Wackermann, Guy, 67700 Monswiller (FR)
(74) Mandataire: Hege, Frédéric
(86) Numéro de dépôt international: PCT/FR2017/050364
(87) Numéro de publication internationale: WO 2017/140995

(56) Documents cités:
- WO-A1-2014/161550
- FR-A1- 2 713 935
- FR-A1- 2 738 481
- US-A1- 2012 297 545
- US-A1- 2014 082 843

## Description

La présente invention se situe dans le domaine de la literie médicale. Elle concerne un matelas pour la prévention et le traitement des escarres.

Les personnes confinées au lit pour des périodes longues sont sujettes aux escarres, des lésions cutanées pouvant aller jusqu'à nécessiter un traitement chirurgical. Le risque est d'autant plus important lorsque les sujets souffrent d'incontinence urinaire. En effet il est important de garder une bonne hygiène de la peau afin d'empêcher la macération, propice au développement des escarres.

La prévention et le traitement des escarres requièrent un nettoyage et une stérilisation fréquents des matelas. Dans le document FR2713935, l'inventeur de la présente demande a proposé un dispositif comprenant une boîte à placer sous la personne alitée, munie d'une composition absorbante et désinfectante. Mais ce dispositif peut facilement se déplacer lorsque le patient bouge et ne plus se trouver à même de recueillir la totalité des urines et des fèces. De plus, cette solution n'est pas confortable pour la personne alitée.

La présente invention a pour objet de pallier au moins en partie à ces inconvénients. A cet effet, elle propose un élément d'absorption et de désinfection, utilisable dans un matelas pour la prévention et le traitement des escarres, comportant un compartiment d'absorption comportant une composition absorbante et un compartiment de désinfection, comportant une composition désinfectante, caractérisé en ce qu'il comporte un film hydrosoluble séparant ledit compartiment d'absorption dudit compartiment de désinfection.

Grâce à ces dispositions, les fluides corporels tels que fèces, urines, sueurs et autres sont absorbés et désinfectés par l'élément d'absorption et de désinfection. L'hygiène de la peau de la personne alitée est donc préservée, empêchant notamment la macération, et les escarres peuvent être évités et/ou traités. Selon d'autres caractéristiques :
- ledit compartiment de désinfection, peut être divisé en une pluralité de sous-compartiments étanches les uns par rapport aux autres ; ainsi lors du transport ou du stockage en position verticale de l'élément d'absorption et de désinfection, la répartition de la composition désinfectante reste suffisamment uniforme et les capacités de désinfection de l'élément d'absorption et de désinfection sont préservées dans l'ensemble du compartiment de désinfection,
- ledit compartiment de désinfection peut comporter au moins un, de préférence deux renforts, permettant un meilleur support mécanique des fesses de la personne alitée.

La présente invention concerne également un matelas d'aide à la prévention et au traitement des escarres comportant un corps principal et un élément échangeable, ledit élément échangeable comportant une composition absorbante et une composition désinfectante.

Grâce à ces dispositions, les fluides corporels tels que fèces, urines, sueurs et autres sont absorbés et désinfectés par les éléments échangeables. L'hygiène de la peau de la personne alitée est donc préservée, empêchant notamment la macération, et les escarres peuvent être évités et/ou traités.

Selon d'autres caractéristiques :
- ledit matelas peut comporter trois éléments échangeables placés selon un axe médian longitudinal aux deux extrémités et au centre du matelas, permettant de couvrir les zones du corps humain diffusant la presque totalité des fluides corporels,
- ledit élément échangeable peut comporter une structure en carton nids d'abeilles alvéolé ou en mousse alvéolée, constituant un bassin d'accueil pour les compositions absorbante et désinfectante,
- ledit matelas peut comporter un film hydrosoluble séparant ladite composition absorbante de ladite composition désinfectante, afin d'éviter que les compositions absorbante et désinfectante ne se mélangent pendant le transport dudit élément échangeable,
- ladite composition absorbante peut comporter un absorbant végétal, en particulier de la rafle de maïs, et se présenter sous forme de granulés, ce qui permet de rendre la composition absorbante plus efficace et plus facile à manipuler et biodégradable,
- ladite composition désinfectante peut contenir du perborate de sodium, permettant d'obtenir un désinfectant peu cher et efficace par hydrolyse,
- ladite composition désinfectante peut comporter en outre un activateur, par exemple un TAED, permettant d'activer l'hydrolyse à une température plus basse, et donc d'améliorer l'efficacité de la désinfection,
- ladite composition désinfectante peut contenir un ampholyte, en particulier de la bétaïne, afin d'accélérer la réaction produisant le désinfectant par hydrolyse du perborate de sodium,
- ledit élément échangeable peut comporter une enveloppe, en particulier en textile non-tissé perméable, constituant un moyen simple et robuste de réalisation de l'invention, et permettant de maintenir l'élément échangeable,
- ledit corps principal peut comporter une housse en jersey plastifié, ce qui permet de protéger le corps principal et de ne pas avoir à le stériliser trop souvent.

La présente invention concerne enfin un procédé de fabrication d'un matelas selon l'invention, comportant les étapes suivantes :
- réalisation du corps principal, revêtu d'une housse, en particulier en jersey plastifié, avec un logement pour un élément échangeable,
- réalisation d'un élément échangeable en carton alvéolé nids d'abeilles ou en mousse alvéolée viscoélastique,
- dépôt d'une composition absorbante dans l'élément échangeable,
- dépôt d'une composition désinfectante sur la composition absorbante,
- disposition d'une enveloppe autour de l'élément échangeable, constituée en particulier d'un textile non-tissé perméable,
- placement de l'élément échangeable dans le logement correspondant du corps principal.

Grâce à ces dispositions, un matelas selon l'invention peut être facilement fabriqué, à partir de matières d'usage classique dans le domaine de la literie hospitalière.

La présente invention sera mieux comprise à la lecture de la description détaillée qui fait suite, en référence aux figures annexées dans lesquelles :
- La figure 1 est une vue en perspective d'un matelas selon un mode préféré de réalisation de l'invention.
- La figure 2 est une vue en coupe d'un élément échangeable selon un mode préféré de réalisation de l'invention.
- La figure 3 est une vue du dessus de la partie principale d'une housse selon un mode préféré de réalisation de l'invention,
- La figure 4 est une vue du dessus d'une deuxième partie d'une housse selon un mode préféré de réalisation de l'invention.
- La figure 5 est une vue du dessus d'une troisième partie d'une housse selon un mode préféré de réalisation de l'invention.
- La figure 6 est une vue en perspective d'un élément échangeable selon un mode préféré de réalisation de l'invention.
- La figure 7 est une vue en perspective d'un élément échangeable selon un autre mode préféré de réalisation de l'invention.

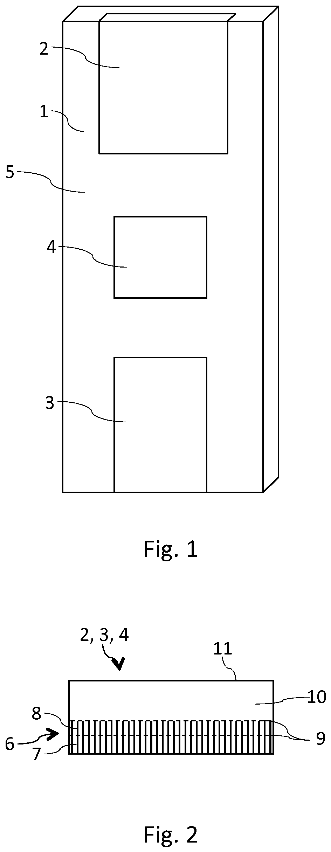

Le matelas selon l'invention comprend un corps principal 1, et un élément échangeable. Dans un mode préféré de réalisation représenté en fig. 1, trois éléments échangeables sont utilisés. Ils sont placés selon un axe médian longitudinal aux deux extrémités et au centre du matelas. Ainsi l'élément échangeable supérieur 2 peut correspondre à la place de la tête et des épaules, l'élément échangeable inférieur 3 peut correspondre à la place des pieds de la personne alitée, et l'élément échangeable central 4 est à même de recueillir les urines et les fèces.

Différentes types de matelas peuvent être envisagés sans sortir du cadre de la présente invention, simplement en adaptant les dimensions du corps principal 1 et de l'élément échangeable 2, 3, 4, ainsi que le placement et le nombre d'éléments échangeables 2, 3, 4. Des exemples d'applications peuvent être, mais ne se limitent pas aux matelas pour lits hospitalisés, tables d'opérations, supports pour grabataires, matelas pour enfants, matelas pour couveuses.

Le corps principal 1 peut comprendre des logements afin d'accueillir les éléments échangeables 2, 3, 4, afin que les éléments échangeables 2, 3, 4 puissent s'insérer dans le corps principal 1, et que l'ensemble du matelas soit sensiblement plat sur sa face supérieure. Ainsi les éléments échangeables 2, 3, 4 sont bloqués en position et le confort pour la personne alitée est proche du confort d'un matelas classique.

Le corps principal 1 peut être recouvert d'une housse 5, celle-ci étant réalisée de sorte à épouser la forme des logements présents dans le corps principal 1 afin d'accueillir les éléments échangeables 2, 3, 4.

Le corps principal 1 peut être réalisé en mousse viscoélastique, ou en toute autre matière habituellement utilisée pour fabriquer des matelas. La housse 5 peut être réalisée en jersey plastifié, connu sous le nom de Dartex (marque déposée).

La fig. 2 représente le détail de l'intérieur d'un élément échangeable 2, 3, 4 selon un mode préféré de réalisation de l'invention. L'élément échangeable 2, 3, 4, ou élément d'absorption et de désinfection, comporte un module d'absorption et de désinfection 6, avec un compartiment d'absorption 7 comportant une composition absorbante 7 et un compartiment de désinfection 8 comportant une composition désinfectante 8. Dans le mode de réalisation de la fig. 2, un film hydrosoluble 9 est placé entre la composition absorbante 7 et la composition désinfectante 8 afin de s'assurer que les compositions absorbante 7 et désinfectante 8 restent en place et ne se mélangent pas durant le transport des éléments échangeables 2, 3, 4. Dès mise en place de l'élément échangeable dans le matelas 1, le premier écoulement de l'urine atteint le film hydrosoluble 9 et le dissout. Ainsi l'urine atteint la composition absorbante, où elle est absorbée.

L'élément échangeable 2, 3, 4 peut encore comporter, au-dessus du module d'absorption et de désinfection 6, un dessus obturant 10. Enfin, le module d'absorption et de désinfection 6 et le dessus obturant 10 de l'élément échangeable 2, 3, 4 peuvent être enveloppés ensemble dans une enveloppe 11.

Le module d'absorption et de désinfection 6 peut être réalisé par exemple en carton nids d'abeille alvéolé ou en mousse alvéolée, par exemple une mousse haute résilience de 80 kg/m3, dite HR 80. L'utilisation du carton nids d'abeille alvéolé résulte en un élément échangeable 2, 3, 4 plus ferme. Il peut donc être judicieux de choisir le carton nids d'abeille alvéolé pour l'élément échangeable central 4 situé au centre du matelas, là ou le matelas est habituellement plus mou, et la mousse alvéolée pour les éléments échangeables 2, 3 situés aux extrémités du matelas.

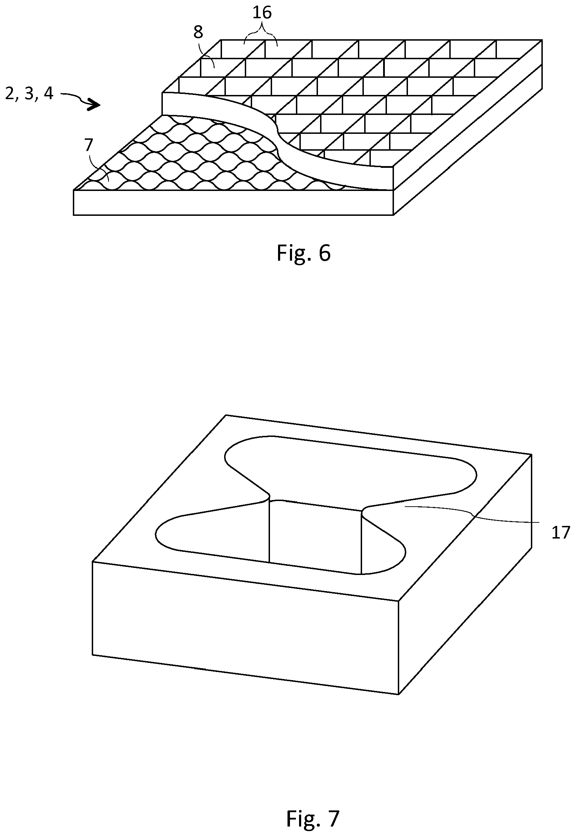

Dans un mode de réalisation représenté en fig. 6, le compartiment de désinfection 8, est divisée en une pluralité de sous-compartiments 16 étanches les uns par rapport aux autres. Ces sous-compartiments permettent que lors de la manutention de l'élément échangeable 2, 3, 4, par exemple lors de son transport ou encore lors de son stockage en position verticale, la répartition de la composition désinfectante 8 reste suffisamment uniforme sur l'ensemble du compartiment de désinfection 8. En particulier, ceci permet que l'entièreté du compartiment de désinfection 8, une fois placé dans un corps principal 1 et en usage, conserve ses capacités de désinfection.

Dans un autre mode de réalisation représenté en fig. 7, le compartiment de désinfection 8 peut comporter au moins un, de préférence deux renforts structurels 17, par exemple en carton, permettant un meilleur support mécanique des fesses de la personne alitée. Ces renforts réduisent la section disponible pour la composition désinfectante 8, qui reste cependant présente en quantité suffisante. De manière avantageuse le compartiment d'absorption 7 peut s'étendre sur toute la section de l'élément échangeable 2, 3, 4, en particulier dans le cas où la composition absorbante 7 comporte de la rafle de maïs, qui, tout en étant peu coûteuse, est suffisamment consistante pour résister à la compression, et soutenir le corps de la personne alitée. Ainsi les renforts 17 présentent une épaisseur réduite à celle du compartiment de désinfection 8, ce qui permet plus facilement de leur donner une résistance mécanique suffisante que s'ils présentaient une épaisseur de la somme des deux compartiments.

Le dessus obturant 10 peut être fait également d'une mousse HR 80. Cette mousse comporte des cellules ouvertes, et elle est privilégiée pour son inertie chimique et micro-organique d'une part, et pour sa souplesse au contact d'autre part. Elle évite au sujet alité les points de pressions durs qui occasionnent rougeurs et escarres. L'enveloppe 11 peut être réalisé en textile non-tissé perméable.

La composition absorbante 7 peut comprendre un composé végétal, par exemple de la rafle de maïs telle que commercialisée par la société Eurocob (marque déposée). Du charbon actif, à la capacité absorbante très importante, peut être ajouté au composé absorbant si l'élément échangeable doit être utilisé pour une longue durée.

Dans un mode préféré de réalisation de l'invention, la composition désinfectante 8 comporte du perborate de sodium tétra hydraté pulvérulent. Au contact d'eau, le perborate de sodium s'hydrolyse afin de libérer de l'acide peracétique qui est un biocide désinfectant. Afin d'activer la formation de l'acide peracétique, la composition désinfectante 8 peut également comporter un activateur tel que du tétraacétyléthylènediamine, aussi appelé TAED, qui permet que l'hydrolyse commence à température plus basse. Enfin, la composition désinfectante 8 peut également comporter un ampholyte tel que la bétaïne, dont le rôle est d'accélérer la réaction. La stérilisation est donc automatique, par une action germicide systématique et totale. Le dosage de la composition désinfectante 8, en particulier les concentrations du perborate de sodium, du TAED et de la bétaïne peuvent être adaptées en fonction de l'usage fait de l'élément échangeable 2, 3, 4.

Les compositions absorbante 7 et désinfectante 8 telles que décrites dans leurs différentes variantes aux paragraphes précédents peuvent être utilisées également dans le cadre d'un élément d'absorption et de désinfection en dehors d'un matelas selon l'invention, par exemple posé sur un matelas. Le confort est moins bon pour le patient, mais l'efficacité d'absorption et de désinfection est similaire.

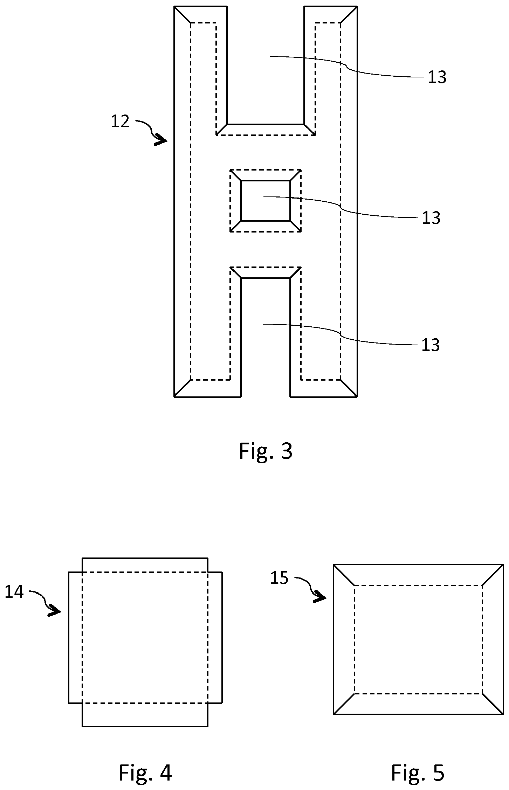

La réalisation d'un matelas peut comporter les étapes suivantes :
- réalisation du corps principal 1, par exemple en mousse viscoélastique, avec un logement pour un élément échangeable 2, 3, 4,
- réalisation d'une housse 5 en jersey plastifié épousant la forme du corps principal 2. Comme représenté à la fig. 3, la housse 5 peut comprendre une partie principale 12 comprenant des ouvertures 13 au niveau des éléments échangeables 2, 3, 4. Comme représenté à la fig. 4, la housse 5 peut comprendre une deuxième et une troisième parties 14, 15 correspondant aux éléments échangeables 2, 3 et 4, qui sont à coller ou coudre dans les ouvertures de la partie principale.
- réalisation d'un élément échangeable 2, 3, 4 en carton alvéolé nids d'abeilles ou en mousse alvéolée viscoélastique ou viscostatique,
- dépôt d'une composition absorbante 7 dans l'élément échangeable 2, 3, 4,
- dépôt d'une composition désinfectante 8 sur la composition absorbante 7,
- disposition d'une enveloppe 11 en textile non-tissé autour de l'élément échangeable 2, 3, 4,
- placement de l'élément échangeable 2, 3, 4 dans le logement correspondant du corps principal 1.

Après utilisation, l'élément échangeable central 4, qui dans un mode préféré de réalisation de l'invention est entièrement composé d'éléments biodégradables, peut être incinéré. Les éléments échangeables 2, 3 peuvent quand à eux être stérilisés et réutilisés. L'invention permet donc d'éviter de stériliser le matelas complet en remplaçant cette étape par un simple remplacement d'un l'élément échangeable 2, 3, 4 à usage unique jetable, et/ou une stérilisation d'un élément échangeable 2, 3, 4, ce qui est plus simple et moins coûteux.

En cas de saturation de l'élément échangeable 2, celui-ci perd sa fonction désinfectante. Il reste néanmoins bactériostatique, c'est-à-dire qu'il ne permet pas au bactéries de se développer, ce qui est essentiel dans la prévention et le traitement des escarres.

## Revendications

1. Elément d'absorption et de désinfection (2, 3, 4), utilisable dans un matelas pour la prévention et le traitement des escarres, comportant un compartiment d'absorption comportant une composition absorbante (7) et un compartiment de désinfection, comportant une composition désinfectante (8), **caractérisé en ce qu'**il comporte un film hydrosoluble (9) séparant ledit compartiment d'absorption (7) dudit compartiment de désinfection (8).

2. Elément (2, 3, 4) selon la revendication précédente, comportant une structure en carton nids d'abeilles alvéolé ou en mousse alvéolée.

3. Elément (2, 3, 4) selon la revendication précédente, dans lequel le compartiment d'absorption (7), comporte une structure en carton nids d'abeilles alvéolé ou en mousse alvéolée.

4. Elément (2, 3, 4) selon l'une des revendications précédentes, dans lequel le compartiment de désinfection (8), est divisé en une pluralité de sous-compartiments (16) étanches les uns par rapport aux autres.

5. Elément (2, 3, 4) selon l'une des revendications précédentes, dans lequel ledit compartiment de désinfection (8) comporte au moins un, de préférence deux renforts (17).

6. Elément (2, 3, 4) selon l'une des revendications précédentes, dans lequel la composition absorbante (7) comporte un absorbant végétal, en particulier de la rafle de maïs, et se présente sous forme de granulés.

7. Elément (2, 3, 4) selon l'une des revendications précédentes, dans lequel la composition désinfectante (8) contient du perborate de sodium.

8. Elément (2, 3, 4) selon l'une des revendications précédentes, dans lequel la composition désinfectante (8) contient un ampholyte, en particulier de la bétaïne.

9. Elément (2, 3, 4) selon l'une des revendications précédentes, comportant une enveloppe (11), en particulier en textile non-tissé perméable.

10. Matelas pour la prévention et le traitement des escarres, comportant un corps principal (1) et au moins un élément (2, 3, 4) selon l'une des revendications précédentes, dans lequel ledit élément (2, 3, 4) est intégré au matelas de manière échangeable.

11. Matelas selon la revendication précédente, comportant trois éléments échangeables (2, 3, 4) placés selon un axe médian longitudinal aux deux extrémités et au centre du matelas.

12. Matelas selon l'une des revendications 10 ou 11, dans lequel le corps principal (1) comporte une housse (5) en jersey plastifié.

13. Procédé de fabrication d'un matelas selon l'une des revendications 10 à 12, comportant les étapes suivantes :
- réalisation du corps principal (1), revêtu d'une housse (5), en particulier en jersey plastifié, avec un logement pour au moins un élément échangeable (2, 3, 4),
- réalisation d'un élément échangeable (2, 3, 4) en carton alvéolé nids d'abeilles ou en mousse alvéolée viscoélastique,
- dépôt d'une composition absorbante (7) dans l'élément échangeable,
- pose d'un film hydrosoluble (9) sur la composition absorbante (7)
- dépôt d'une composition désinfectante (8) sur la composition absorbante,
- disposition d'une enveloppe (11) autour de l'élément échangeable (2, 3, 4), constituée en particulier d'un textile non-tissé perméable,
- placement de l'élément échangeable (2, 3, 4) dans le logement correspondant du corps principal (1).

## Patentansprüche

1. Absorptions- und Desinfektionselement (2, 3, 4), das in einer Matratze zur Vorbeugung und Behandlung von Schorf verwendbar ist, umfassend ein Absorptionsfach, das eine absorbierende Zusammensetzung (7) umfasst, und ein Desinfektionsfach, das eine Desinfektionszusammensetzung (8) umfasst, **dadurch gekennzeichnet, dass** es einen wasserlöslichen Film (9) umfasst, der das Absorptionsfach (7) von dem Desinfektionsfach (8) trennt.

2. Element (2, 3, 4) nach dem vorstehenden Anspruch, umfassend eine Wabenwellpappe oder Wabenschaum-struktur.

3. Element (2, 3, 4) nach dem vorstehenden Anspruch, bei dem der Absorptionsraum (7) eine Wabenwellpappe- oder Wabenschaumstruktur aufweist.

4. Element (2, 3, 4) nach einem der vorstehenden Ansprüche, bei dem das Desinfektionsfach (8) in eine Vielzahl von Unterabteilungen (16) unterteilt ist, die gegeneinander wasserdicht sind.

5. Element (2, 3, 4) nach einem der vorhergehenden Ansprüche, wobei das Desinfektionsfach (8) mindestens eine, vorzugsweise zwei Verstärkungen (17) umfasst.

6. Element (2, 3, 4) nach einem der vorstehenden Ansprüche, wobei die absorbierende Zusammensetzung (7) ein pflanzliches Absorptionsmittel, insbesondere Maiskolben, umfasst und in Form von Granulat vorliegt.

7. Element (2, 3, 4) nach einem der vorstehenden Ansprüche, worin die Desinfektionsmittelzusammensetzung (8) Natriumperborat enthält.

8. Element (2, 3, 4) nach einem der vorstehenden Ansprüche, worin die Desinfektionszusammensetzung (8) ein Ampholyt, insbesondere Betain, enthält.

9. Element (2, 3, 4) nach einem der vorstehenden Ansprüche, umfassend eine Hülle (11), insbesondere aus permeablem Vliesstoff.

10. Matratze zur Vorbeugung und Behandlung von Schorf, umfassend einen Hauptkörper (1) und mindestens ein Element (2, 3, 4) nach einem der vorstehenden Ansprüche, wobei das Element (2, 3, 4) in die Matratze austauschbar integriert ist.

11. Matratze nach dem vorherigen Anspruch, umfassend drei austauschbare Elemente (2, 3, 4), die entlang einer Längsmittellinie an beiden Enden und in der Mitte der Matratze angeordnet sind.

12. Matratze nach einem der Ansprüche 10 oder 11, wobei der Hauptkörper (1) eine Schutzhülle (5) aus plastifiziertem Jersey aufweist.

13. Verfahren zur Herstellung einer Matratze nach einem der Ansprüche 10 bis 12, umfassend die folgenden Schritte:
- Herstellung des Hauptkörpers (1), der mit einer Schutzhülle (5), insbesondere aus plastifiziertem Jersey, überzogen ist, mit einem Gehäuse für mindestens ein austauschbares Element (2, 3, 4),
- Herstellung eines austauschbaren Elements (2, 3, 4) aus einer viskoelastischen Wabenwellpappe oder Wabenschaum-struktur,
- Einreichung einer absorbierenden Zusammensetzung (7) in das austauschbare Element,
- Aufbringen eines wasserlöslichen Films (9) auf die absorbierende Zusammensetzung (7)
- Einreichung einer Desinfektionsmittelzusammensetzung (8) auf die absorbierende Zusammensetzung,
- Anordnung einer Hülle (11) um das austauschbare Element (2, 3, 4), bestehend insbesondere aus einem permeablen Vliesstoff,
- Einsetzen des austauschbaren Elements (2, 3, 4) in das entsprechende Gehäuse des Hauptkörpers (1).

## Claims

1. Absorption and disinfection element (2, 3, 4), usable in a mattress for the prevention and treatment of bedsores, comprising an absorption compartment comprising an absorbent composition (7) and a disinfection compartment, comprising a disinfecting composition (8), **characterized in that** it comprises a water-soluble film (9) separating said absorption compartment (7) from said disinfection compartment (8).

2. Element (2, 3, 4) according to the previous claim, comprising a cardboard honeycomb or a cellular foam structure.

3. Element (2, 3, 4) according to the previous claim, wherein the absorption compartment (7) comprises a cardboard honeycomb or a cellular foam structure.

4. Element (2, 3, 4) according to one of the previous claims, wherein the disinfection compartment (8) is divided into a plurality of sub-compartments (16) which are watertight with respect to each other.

5. Element (2, 3, 4) according to one of the previous claims, wherein said disinfection compartment (8) comprises at least one, preferably two reinforcements (17).

6. Element (2, 3, 4) according to one of the previous claims, wherein the absorbent composition (7) comprises a vegetable absorbent, in particular corn cob, and is in the form of granules.

7. Element (2, 3, 4) according to one of the previous claims, wherein the disinfectant composition (8) contains sodium perborate.

8. Element (2, 3, 4) according to one of the previous claims, wherein the disinfectant composition (8) contains an ampholyte, in particular betaine.

9. Element (2, 3, 4) according to one of the previous claims, comprising an envelope (11), in particular made of permeable nonwoven textile.

10. A mattress for preventing and treating bedsores, comprising a main body (1) and at least one element (2, 3, 4) according to one of the previous claims, wherein said element (2, 3, 4) is integrated in the mattress in an exchangeable manner.

11. Mattress according to the previous claim, comprising three exchangeable elements (2, 3, 4) placed along a longitudinal median axis at both ends and in the center of the mattress.

12. Mattress according to one of claims 10 or 11, wherein the main body (1) has a cover (5) made of plasticized jersey.

13. A method for manufacturing a mattress according to one of claims 10 to 12, comprising the following steps:
- production of the main body (1), covered with a cover (5), in particular in plasticised jersey, with a housing for at least one exchangeable element (2, 3, 4),
- production of an exchangeable element (2, 3, 4) in honeycombed cardboard or viscoelastic cellular foam,
- deposition of an absorbent composition (7) in the exchangeable element,
- application of a water-soluble film (9) onto the absorbent composition (7)
- deposition of a disinfectant composition (8) on the absorbent composition,
- arrangement of an envelope (11) around the exchangeable element (2, 3, 4), made in particular of a permeable nonwoven textile,
- placement of the exchangeable element (2, 3, 4) in the corresponding housing of the main body (1).
